# FASCICULE DE BREVET EUROPEEN

(11) **EP 1 039 352 B1**
(45) Date de publication et mention de la délivrance du brevet: **08.10.2003**
(21) Numéro de dépôt: 99810263.6
(22) Date de dépôt: 26.03.1999
(51) Int. Cl.: G04B 17/22

(54) **Spiral autocompensateur pour balancier-spiral de mouvement d'horlogerie et procédé de traitement de ce spiral**
Selbstkompensierende Spiralfeder für Uhrwerkspiralfederunruh und Verfahren zur Behandlung derselben
Self-compensating spring for clockwork movement spring balance and method for treating the same

(43) Date de publication de la demande: 27.09.2000
(73) Titulaire: ROLEX SA, 1211 Genève 24 (CH); Manufacture des Montres Rolex S.A., 2502 Bienne (CH)
(72) Inventeur: Baur, Jacques, 1170 Aubonne (CH); Sol, Patrick, 01420 Chanay (FR)
(74) Mandataire: Savoye, Jean-Paul

(56) Documents cités:
- EP-A- 0 886 195
- CH-A- 340 777
- CH-A4- 1 508 166
- US-A- 4 765 335

## Description

La présente invention se rapporte à un spiral autocompensateur pour oscillateur mécanique balancier-spiral de mouvement d'horlogerie ou autre instrument de précision, en alliage paramagnétique contenant au moins un des éléments Nb, V, Ta, Ti, Zr, Hf, ainsi qu'à un procédé de traitement de ce spiral.

L'existence de phénomènes de dérive de la fréquence d'un oscillateur balancier-spiral en fonction du temps est bien connue des horlogers. C'est ainsi qu'un tel oscillateur muni d'un spiral brut de fabrication en alliage ferromagnétique va progressivement voir sa fréquence augmenter pour atteindre au bout d'un an une variation de marche de l'ordre de 10 s/jour.

Ceci s'explique essentiellement par le fait que les opérations de fabrication génèrent des perturbations dans la structure cristalline du spiral. Par exemple, les déformations plastiques résultant du tréfilage, du laminage ou du formage de la courbe Breguet créent des défauts du cristal comme des lacunes, des interstitiels ou des dislocations. Des refroidissements trop rapides après traitement thermique piègent des lacunes. Des contraintes internes sont également générées par les déformations plastiques ou les brusques variations de température. De l'oxygène peut diffuser dans le cristal lors des traitements thermiques.

L'ensemble de ces perturbations de la structure cristalline induisent des phénomènes anélastiques qui modifient légèrement le module de Young du spiral. Usuellement, les phénomènes anélastiques conduisent à un module plus faible que le module purement élastique.

Au cours du temps, les défauts créés dans la structure cristalline vont migrer par diffusion lente à température ambiante, vers des positions d'équilibre plus stables et les contraintes internes vont se relaxer. Ces mécanismes de réorganisation de la structure vont ainsi faire disparaître lentement les phénomènes anélastiques qui perturbaient le module de Young. En général, on observe une augmentation du module qui va tendre vers sa valeur purement élastique.

Les variations relatives dues aux phénomènes anélastiques et à leur disparition sont extrêmement faibles, de l'ordre de 10⁻⁴, mais néanmoins clairement mesurables sur la marche d'une montre, puisqu'une variation relative du module de 2,3x10⁻⁵ correspond à une variation de marche de 1 s/jour.

Afin de réduire cette dérive, on effectue habituellement des traitements thermiques, appelés traitements d'étuvage, qui consistent à chauffer les spiraux terminés à une température modérée comprise entre 100° et 250°C pendant une durée de 6 à 24h. Ces traitements permettent de réduire la dérive de marche au cours des premières années au-dessous de 1 s/jour, ce qui est acceptable, compte tenu des autres perturbations dues au porter de la montre, comme les chocs. L'étuvage a pour effet d'accélérer par activation thermique le processus de relaxation des contraintes et de diffusion des défauts vers leurs positions d'équilibre.

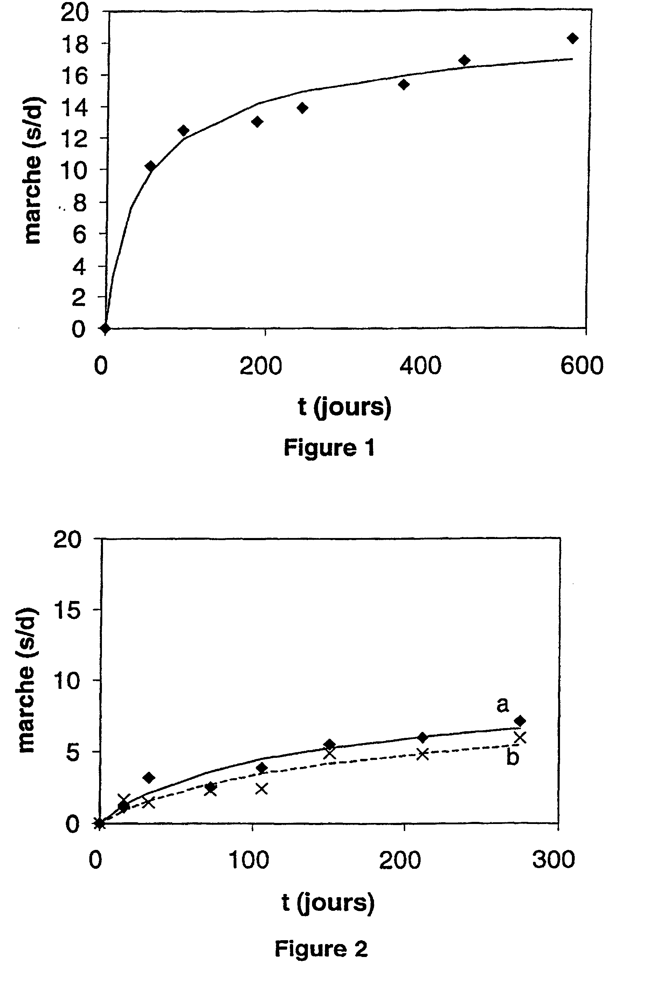

On constate le même phénomène de dérive dans le cas de spiraux en alliages paramagnétiques, notamment Nb-Zr, bien que plus accentué encore, comme illustré par le diagramme de la figure 1. Contrairement à ce qui se passe pour les spiraux en alliages ferromagnétiques, dans le cas d'un tel spiral en alliage paramagnétique, le même type de traitement d'étuvage ne permet pas de réduire la dérive de marche au-dessous d'environ 5 s/jour au bout d'un an, comme le montre le diagramme de la figure 2 où la courbe a) correspond à un spiral traité à 170°C et la courbe b) à un spiral traité à 270°C.

L'impossibilité de réduire dans des limites acceptables, voire d'éliminer la dérive de marche résiduelle d'oscillateurs balancier-spiral équipés de spiraux en alliage paramagnétique, notamment Nb-Zr, peut faire penser que d'autres mécanismes modifient le couple exercé par le spiral, en plus de ceux décrits précédemment pour les spiraux en alliages ferromagnétiques.

Les alliages paramagnétiques ont une très grande affinité pour l'oxygène. A l'air ambiant, il se forme un film d'oxyde en surface qui passive l'alliage. La présence de ce film, malgré sa faible épaisseur de quelques nm, perturbe le couple exercé par le spiral. En effet, l'épaisseur du spiral est d'environ 30 à 50 µm et les variations relatives de couple qui perturbent la marche de l'oscillateur sont de l'ordre de 10⁻⁴, soit l'ordre de grandeur du rapport d'épaisseur du film d'oxyde à l'épaisseur du spiral. Ces considérations suggèrent qu'une part des dérives de marche observées avec les spiraux en Nb-Zr sont dues à la modification du film d'oxyde à l'air ambiant au cours du temps, ce qui explique la raison pour laquelle le traitement d'étuvage classique ne permet pas de résoudre à lui seul ce problème de dérive, comme il permet de le faire dans le cas des spiraux en alliages ferromagnétiques.

Ainsi, il est possible de déduire de ces considérations que la dérive de marche d'un balancier-spiral équipé d'un spiral en alliage paramagnétique, notamment en alliage Nb-Zr est due à la sommes de deux effets:
a) un effet de volume créé par la réorganisation lente de la microstructure à température ambiante. Cet effet est similaire à ce qui est observé pour les spiraux ferromagnétiques et peut être éliminé par étuvage;
b) un effet de surface créé par l'oxydation et la passivation de la couche superficielle au contact de l'air.

Le but de la présente invention est de réduire, voire de supprimer, la dérive de marche due à l'effet b) susmentionné.

A cet effet, cette invention a pour objet un spiral autocompensateur pour oscillateur mécanique balancier-spiral de mouvement d'horlogerie ou autre instrument de précision, selon la revendication 1. Cette invention a également pour objet un procédé de traitement de ce spiral selon la revendication 4.

La croissance d'une couche d'oxyde à la surface du spiral sert également à le passiver, de sorte qu'il ne subira plus de dérive de son couple au cours du temps, due à la formation lente de la couche d'oxyde à l'air libre, comme ceci se produit autrement. L'avantage du procédé d'anodisation pour former cette couche d'oxyde est de pouvoir se dérouler à basse température sans interférer avec la structure cristalline du spiral. De plus ce traitement par anodisation permet de régler avec une très grande simplicité et en toute sécurité et avec une parfaite reproductibilité l'épaisseur de la couche d'oxyde à la valeur désirée. En outre, l'épaisseur de cette couche et donc la couleur du spiral ainsi revêtu est d'une parfaite régularité.

D'autres avantages de la présente invention apparaîtront à la lumière de la description ainsi que des diagrammes explicatifs annexés, relatifs à une forme d'exécution de l'invention, donnée à titre d'exemple.
La figure 1 est un diagramme de l'évolution de la dérive journalière en s/jour en fonction du temps t en jours d'une montre dont le balancier-spiral est muni d'un spiral Nb-Zr 18%, brut de fabrication;
la figure 2 est un diagramme semblable à celui de la figure 1 pour une montre dont le balancier-spiral est muni d'un spiral Nb-Zr 18% étuvé sous azote durant 24h;
la figure 3 est un même diagramme relatif à deux montres équipées a) d'un spiral Nb-Zr 18% selon l'invention et étuvé, b) d'un même spiral uniquement étuvé.

Selon un mode de mise en oeuvre du procédé objet de la présente invention, on prépare une solution d'acide sulfurique dilué à 0,1% vol. dans de l'eau. On immerge le spiral dans cette solution en faisant passer un courant de faible intensité sous une tension de 30 V pendant 30 s. Le courant de faible intensité permet de ne pas chauffer notablement le spiral pendant le traitement d'anodisation de manière ne pas modifier le coefficient thermique du module de Young (CTE). Dans ces conditions, la couche d'oxyde qui se forme a une épaisseur de l'ordre 50 nm, donnant au spiral une couleur bleue. Ce spiral est ensuite étuvé pendant 6h à 200°C sous atmosphère d'azote. Pour varier la couleur, il suffit de modifier la tension d'anodisation. Une tension donnée correspond à une couleur déterminée. L'arrêt du processus d'anodisation est fonction de l'épaisseur de la couche, elle-même fonction de la tension de claquage de cette couche d'oxyde. Ceci explique le fait que l'épaisseur de la couche et donc sa couleur sont parfaitement contrôlables et reproductibles. De plus, la couleur est homogène sur toute la surface du spiral.

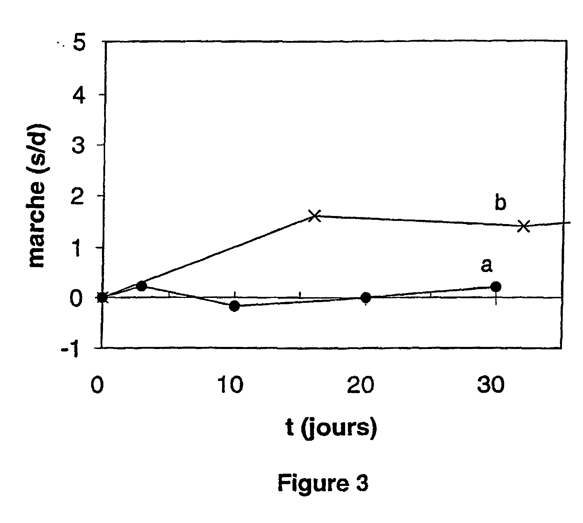

La courbe a) de la figure 3 montre que la dérive journalière d'une montre équipée de ce spiral est pratiquement nulle, contrairement à la courbe b) relative au spiral n'ayant subi qu'un traitement thermique d'étuvage.

L'épaisseur de la couche d'oxyde n'a pas d'importance pour atteindre le but de la présente invention, de sorte que l'on peut choisir cette épaisseur en fonction de la couleur désirée. C'est ainsi que dans les conditions d'anodisation susmentionnées, en appliquant au spiral une tension de 15V, on obtient une couleur jaune et avec une tension de 18V une couleur rouge. En général, l'épaisseur de la couche colorée se situe entre 20 et 200 nm.

Il faut néanmoins prendre en compte le fait que la formation d'une couche d'oxyde de 20 à 200 nm diminue déjà le CTE apparent du spiral d'environ 5ppm/°C et augmente le couple. Il est donc nécessaire de tenir compte de ces différences lors du traitement thermique préalable de fixage de la forme, de manière à obtenir le CTE et le couple recherchés après le traitement d'oxydation par anodisation.

La dérive de la marche consécutive à l'oxydation lente du spiral en Nb-Zr à l'air ambiant est un phénomène très général pour tous les alliages de Nb ou contenant de fortes proportions de Nb, en particulier, lorsque les principaux éléments alliants sont aussi très réactifs à l'oxygène, comme Ti, V, Zr, Cr, Ta, Mo, Hf.

D'une manière encore plus générale, on sait du CH 551 032 que les alliages paramagnétiques à base de Nb, Ta, V sont susceptibles de présenter un CTE positif. Ces éléments sont très réactifs à l'oxygène et aussi susceptibles de créer une dérive du couple du spiral par oxydation de la surface à l'air ambiant.

Le DE 15 58 816 cite encore les alliages à base d'au moins un élément du groupe Nb, V, Ta et un élément du groupe Ti, Zr, Hf comme susceptibles d'avoir un CTE positif. Tous les alliages de ce type sont aussi très réactifs à l'oxygène et créeront une dérive du couple du spiral par oxydation à l'air ambiant, de sorte qu'ils peuvent tous être revêtus d'une couche d'oxyde conformément à l'objet de la présente invention.

L'oxydation thermique serait une autre voie possible, bien que moins favorable que l'anodisation, car elle implique un traitement thermique à relativement haute température. Typiquement, l'obtention d'une couche de couleur bleue s'obtient par traitement de 2 à 3 minutes à 450°C dans l'air. Cette température est suffisamment élevée pour permettre la diffusion de l'oxygène dans le volume du spiral, de sorte que le CTE sera perturbé en plus de l'effet dû à la couche d'oxyde. Aussi, la reproductibilité de ce type de traitement est plus aléatoire que l'anodisation.

## Revendications

1. Spiral autocompensateur pour oscillateur mécanique balancier-spiral de mouvement d'horlogerie ou autre instrument de précision, en alliage paramagnétique contenant au moins un des éléments Nb, V, Ta, Ti, Zr, Hf, **caractérisé en ce qu'**il est recouvert d'une couche sensiblement uniforme d'oxyde d'une épaisseur supérieure ou égale à 20nm.

2. Spiral selon la revendication 1, **caractérisé en ce que** l'épaisseur de ladite couche d'oxyde est apte à le colorer.

3. Spiral selon l'une des revendications précédentes, **caractérisé en ce qu'**il est en un alliage Nb-Zr contenant entre 5% et 25% de Zr.

4. Procédé de traitement d'un spiral autocompensateur selon la revendication 1, **caractérisé en ce que** l'on forme ladite couche d'oxyde sensiblement uniforme en soumettant ledit spiral à un traitement d'anodisation.

5. Procédé selon la revendication 4, **caractérisé en ce que** l'on applique une tension de 10 à 35 V dans le bain d'anodisation, pendant une durée comprise entre 10 et 200 s.

6. Procédé selon l'une des revendications 4 et 5, **caractérisé en ce que** l'on augmente la valeur du CTE du spiral et on abaisse la valeur du couple avant traitement d'anodisation, pour compenser leurs variations consécutives à la formation de ladite couche d'oxyde.

7. Procédé selon l'une des revendications 4 et 5, **caractérisé en ce que**, l'on soumet ledit spiral anodisé à un traitement thermique d'étuvage entre 100° et 250°C pendant 1 à 24h.

## Patentansprüche

1. Selbstkompensierende Spiralfeder für einen mechanischen Spiralfeder-Unruhoszillator eines Uhrwerks oder anderen Präzisionsinstruments aus einer zumindest eines der Elemente Nb, V, Ta, Ti, Zr, Hf enthaltenden paramagnetischen Legierung, **dadurch gekennzeichnet, dass** sie von einer im Wesentlichen gleichförmigen Oxidschicht einer Dicke bedeckt ist, die 20 nm oder mehr beträgt.

2. Spiralfeder nach Anspruch 1, **dadurch gekennzeichnet, dass** die Dicke dieser Oxidschicht geeignet ist, sie zu färben.

3. Spiralfeder nach einem der vorangehenden Ansprüche, **dadurch gekennzeichnet, dass** sie aus einer Nb―Zr-Legierung besteht, die zwischen 5 % und 25 % Zr enthält.

4. Verfahren zur Behandlung einer selbstkompensierenden Spiralfeder nach Anspruch 1, **dadurch gekennzeichnet, dass** man diese im Wesentlichen gleichförmige Oxidschicht bildet, indem man diese Spiralfeder einer Anodisierungsbehandlung unterwirft.

5. Verfahren nach Anspruch 4, **dadurch gekennzeichnet, dass** während einer Zeit zwischen 10 und 200 Sekunden eine Spannung von 10 bis 35 V im Anodisierungsbad angelegt wird.

6. Verfahren nach einem der Ansprüche 4 und 5, **dadurch gekennzeichnet, dass** vor der Anodisierungsbehandlung der Wert des TKE der Spiralfeder erhöht und der Wert der Federkonstante verringert wird, um ihre Veränderungen nach Bildung dieser Oxidschicht zu kompensieren.

7. Verfahren nach einem der Ansprüche 4 und 5, **dadurch gekennzeichnet, dass** man diese anodisierte Spiralfeder während 1 bis 24 Stunden einer Heissbehandlung durch Ausheizen zwischen 100 und 250 °C unterwirft.

## Claims

1. Self-compensating spiral for a mechanical spiral balance-wheel oscillator in watchwork or other precision instrument, made of a paramagnetic alloy containing at least one of the elements Nb, V, Ta, Ti, Zr, Hf, **characterized in that** it is covered with a substantially uniform oxide layer having a thickness greater than or equal to 20 nm.

2. Spiral according to Claim 1, **characterized in that** the thickness of the said oxide layer is capable of coloring it.

3. Spiral according to one of the preceding claims, **characterized in that** it is made of an Nb-Zr alloy containing between 5% and 25% Zr.

4. Process for treating a self-compensating spiral according to Claim 1, **characterized in that** the said substantially uniform oxide layer is formed by subjecting the said spiral to an anodizing treatment.

5. Process according to Claim 4, **characterized in that** a voltage of 10 to 35 V is applied in the anodizing bath for a time of between 10 and 200 s.

6. Process according to either of Claims 4 and 5, **characterized in that** the value of the TCE of the spiral is increased and the value of the torque is reduced before the anodizing treatment, in order to compensate for their variations resulting from the formation of the said oxide layer.

7. Process according to either of Claims 4 and 5, **characterized in that** the said anodized spiral is subjected to an oven heat treatment between 100° and 250°C for 1 to 24 h.
